# EUROPEAN PATENT SPECIFICATION

(11) **EP 4 262 686 B1**
(45) Date of publication and mention of the grant of the patent: **07.01.2026**
(21) Application number: 21840197.4
(22) Date of filing: 10.12.2021
(51) Int. Cl.: A61K 8/26, A61K 8/36, A61K 8/44, A61K 8/73, A61Q 19/10

(54) **COMBINATION OF MODIFIED STARCH/C13-C15 FATTY ACID/CLAY WITH AMPHOTERIC SURFACTANT**
KOMBINATION VON MODIFIZIERTER STÄRKE/C13-C15 FETTSÄURE/TON MIT AMPHOTEREM TENSID
COMBINAISON AMIDON MODIFIÉ/ACIDE GRAS C13-C15/ARGILE AVEC TENSIOACTIF AMPHOTÈRE

(30) Priority: 16.12.2020 JP 2020208439; 20.01.2021 FR 2100546
(43) Date of publication of application: 25.10.2023
(73) Proprietor: L'OREAL, 75008 Paris (FR); Endo, Koji, Kawasaki-shi, Kanagawa, 213-0012 (JP); Mishina, Natsuno, Kawasaki-shi, Kanagawa, 213-0012 (JP); Orita, Masanori, Kawasaki-shi, Kanagawa, 213-0012 (JP)
(72) Inventor: ENDO, Koji, Kawasaki-shi, Kanagawa 2130012 (JP); MISHINA, Natsuno, Kawasaki-shi, Kanagawa 2130012 (JP); ORITA, Masanori, Kawasaki-shi, Kanagawa 2130012 (JP)
(74) Representative: Casalonga
(86) International application number: PCT/JP2021/046654
(87) International publication number: WO 2022/131352

(56) References cited:
- WO-A1-2015/181789
- WO-A1-2020/218044
- US-A1- 2005 233 916
- DATABASE GNPD [online] MINTEL; 18 December 2020 (2020-12-18), ANONYMOUS: "Foam Cleanser", XP055855038, retrieved from https://www.gnpd.com/sinatra/recordpage/8353029/ Database accession no. 8353029

## Description

### TECHNICAL FIELD

The present invention relates to a composition comprising a combination of modified starch, C₁₃-C₁₅ fatty acid, clay, and amphoteric surfactant, as well as a use of the composition.

### BACKGROUND ART

The use of mineral clays as medicinal and cosmetic tools has been popular for a long time.

Clays are used to absorb excess oil, dirt, and toxins from the skin while simultaneously exfoliating and improving skin circulation. Some clays, such as bentonite clay, are primarily ingested for medicinal purposes such as detoxification or mineral deficiencies.

Other clays, such as French Green clay and Rhassoul clay, are used externally for skin conditions and for cosmetic purposes. Clays come in a variety of colors such as red, green, white, gray, and can range in texture from coarse and heavy to fine and fluffy. The different colors of clays occur because of their natural mineral content.

In modern cosmetics, such clays are used as key active ingredients for oil control products which are sold in the form of oil control face washes, etc. In some rinse off cosmetic compositions, clays are included.

### DISCLOSURE OF INVENTION

A rinse off cosmetic composition including clay may leave a deposition of the clay on a keratin substance such as skin even after the composition is rinsed off from the keratin substance. The remaining deposition of clays, if present, can contribute to oil control.

It has been discovered that a combination of modified starch and C₁₃-C₁₅ fatty acid can enhance the deposition of clay on a keratin substance such as skin. Combinations of modified starch, C₁₃-C₁₅ fatty acid and clay are known from WO 2020/218044.

There is still a need to further enhance the deposition of clay on a keratin substance such as skin.

An objective of the present invention is to provide a composition which can be rinsed off from a keratin substance such as skin and can leave an enhanced deposition of clay on the keratin substance after rinsing off the composition from the keratin substance.

The above objective can be achieved by a composition, comprising:
(a) at least one modified starch;
(b) at least one C₁₃₋C₁₅ acid;
(c) at least one clay; and
(d) at least one amphoteric surfactant,
wherein
the weight ratio of the amount of the (d) amphoteric surfactant/the amount of the (b) C₁₃-C₁₅ fatty acid is 0.5 or more, preferably 0.6 or more, and more preferably 0.7 or more.

The (a) modified starch may be hydrophobic, preferably hydroxyalkyl-modified starch, and more preferably selected from the group consisting of hydroxyethyl starch, hydroxypropyl starch, hydroxyethyl starch phosphate, hydroxypropyl starch phosphate, and a mixture thereof.

The amount of the (a) modified starch in the composition according to the present invention may be from 0.01% to 15% by weight, preferably from 0.1% to 10% by weight, and more preferably from 0.5% to 5% by weight, relative to the total weight of the composition.

The (b) C₁₃-C₁₅ fatty acid may be myristic acid.

The amount of the (b) C₁₃-C₁₅ fatty acid in the composition according to the present invention may be from 1% to 20% by weight, preferably from 2% to 15% by weight, and more preferably from 3% to 10% by weight, relative to the total weight of the composition.

The (c) clay may be selected from the group consisting of hectorite, kaolin, talc, and a mixture thereof.

The amount of the (c) clay in the composition according to the present invention may be from 1% to 40% by weight, preferably from 2% to 35% by weight, and more preferably from 3% to 30% by weight, relative to the total weight of the composition.

The (a) modified starch and the (b) C₁₃-C₁₅ fatty acid may form a complex.

The (c) clay may be coated with the (a) modified starch and the (b) C₁₃-C₁₅ fatty acid, preferably a complex formed by the (a) modified starch and the (b) C₁₃-C₁₅ fatty acid, and more preferably a complex formed by hydrophobic modified starch and myristic acid.

The (d) amphoteric surfactant may be selected from betaines, amine oxides and mixtures thereof.

The amount of the (d) amphoteric surfactant in the composition according to the present invention may be from 1% to 20% by weight, preferably from 2% to 15% by weight, and more preferably from 3% to 10% by weight, relative to the total weight of the composition.

The composition according to the present invention may further comprise (e) water.

The pH of the composition according to the present invention may be more than 7.0, preferably more than 7.5, and more preferably more than 8.0.

The composition according to the present invention may be a cosmetic composition, preferably a rinse-off composition, and more preferably a rinse-off cleansing composition.

The present invention also relates to a cosmetic process for a keratin substance, such as skin, comprising the step of:
applying the composition according to the present invention onto the keratin substance.

The present invention also relates to a use of a combination of:
(a) at least one modified starch;
(b) at least one C₁₃-C₁₅ fatty acid; and
(d) at least one amphoteric surfactant,
wherein
the weight ratio of the amount of the (d) amphoteric surfactant/the amount of the (b) C₁₃-C₁₅ fatty acid is 0.5 or more, preferably 0.6 or more, and more preferably 0.7 or more,
in order to increase the deposition on a keratin substance such as skin of (c) at least one clay.

### BRIEF DESCRIPTION OF DRAWINGS

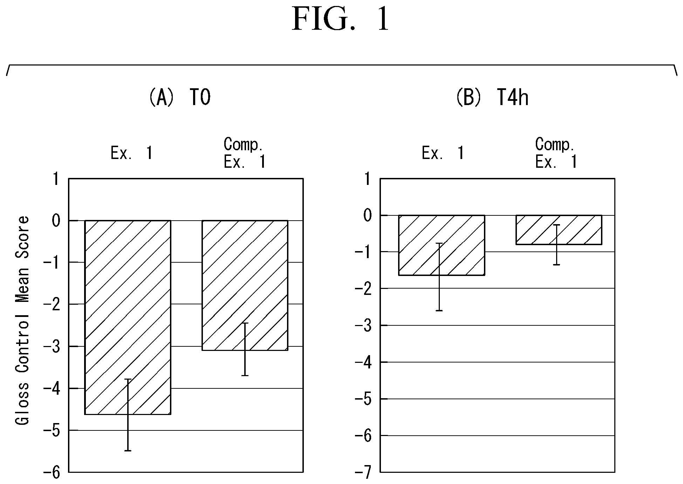

Figure 1 shows a graph showing the gloss control by the compositions according to Example 1 and Comparative Example 1, just after (T0) the application of each composition (A) and 4 hours after (T4h) the application (B).

### BEST MODE FOR CARRYING OUT THE INVENTION

After diligent research, the inventors have discovered that it is possible to provide a composition which can be rinsed off from a keratin substance such as skin and can leave an enhanced deposition of clay on the keratin substance after rinsing off the composition from the keratin substance.

The composition according to the present invention comprises a combination of (a) at least one modified starch, (b) at least one C₁₃-C₁₅ fatty acid, (c) at least one clay, and (d) at least one amphoteric surfactant, under certain conditions.

The composition according to the present invention can be rinsed off from a keratin substance such as skin and can leave an increased amount of a deposition of clay on the keratin substance after rinsing off the composition from the keratin substance.

A deposition of clay can contribute to oil control. Since the composition according to the present invention can increase the amount of the deposition of clay on a keratin substance such as skin, the composition according to the present invention can provide immediate and long-lasting oil control effects. Thus, for example, the composition according to the present invention can control or suppress immediately and for a long period of time non-preferable events such as greasy appearance caused by oily substances such as sebum on a keratin substance such as skin. Also, the increased amount of the deposition of clay on a keratin substance such as skin can provide a smooth feeling to touch immediately and for a long period of time.

The (c) clay may be coated with the (a) modified starch and the (b) C₁₃-C₁₅ fatty acid. The (a) modified starch and the (b) C₁₃-C₁₅ fatty acid may form a complex. Thus, the (c) clay may be coated with a complex formed by the (a) modified starch and the (b) C₁₃-C₁₅ fatty acid. It is preferable that the complex be formed by hydrophobic modified starch and myristic acid.

The hydrophobicity of the (c) clay can be enhanced by the (a) modified starch and the (b) C₁₃-C₁₅ fatty acid, preferably a complex formed by the (a) modified starch and the (b) C₁₃-C₁₅ fatty acid, and more preferably a complex formed by hydrophobic modified starch and myristic acid. Therefore, the (c) clay can deposit more on a keratin substance such as skin, due to hydrophobic-hydrophobic interaction between the (c) clay and the keratin substance. This can result in the increase in the amount of the deposition of the (c) clay on the keratin substance.

Furthermore, surprisingly, the additional use of the (d) amphoteric surfactant can further enhance the deposition of the (c) clay on the keratin substance.

Without being bound by theory, it is believed that the cationic moieties of the (d) amphoteric surfactant can reduce the negative charges of a possible complex of the (a) modified starch and the (b) C₁₃-C₁₅ fatty acid on the (c) clay which could be attributed to the (b) C₁₃-C₁₅ fatty acid, and therefore, the (c) clay with the possible complex having reduced negative charges can be relatively easily absorbed on the keratin substance, such as skin, which inherently has negative charges.

Hereafter, the present invention will be described in a detailed manner.

### [Composition]

One of the aspects of the present invention is a composition, comprising:
(a) at least one modified starch;
(b) at least one C₁₃-C₁₅ fatty acid;
(c) at least one clay; and
(d) at least one amphoteric surfactant,
wherein
the weight ratio of the amount of the (d) amphoteric surfactant/the amount of the (b) C₁₃-C₁₅ fatty acid is 0.5 or more, preferably 0.6 or more, and more preferably 0.7 or more.

In one embodiment of the present invention,
the amount of the (a) modified starch in the composition is from 0.01% to 15% by weight, preferably from 0.1% to 10% by weight, and more preferably from 0.5% to 5% by weight, relative to the total weight of the composition,
the amount of the (b) C₁₃-C₁₅ fatty acid is from 1% to 20% by weight, preferably from 2% to 15% by weight, and more preferably from 3% to 10% by weight, relative to the total weight of the composition,
the amount of the (c) clay is from 1% to 40% by weight, preferably from 5% to 35% by weight, and more preferably from 10% to 30% by weight, relative to the total weight of the composition, and
the amount of the (d) amphoteric surfactant in the composition according to the present invention may be from 1% to 20% by weight, preferably from 2% to 15% by weight, and more preferably from 3% to 10% by weight, relative to the total weight of the composition.

The composition according to the present invention may further comprise (e) water.

### (Modified Starch)

The composition according to the present invention comprises (a) at least one modified starch. A single type of modified starch may be used, or two or more different types of modified starches may be used in combination.

The (a) modified starch may be in the form of a powder. In other words, the (a) modified starch may be in the form of particles. In this case, the particle size of the (a) modified starch is not limited.

It is preferable that the (a) modified starch is film-forming, i.e., is capable of forming a film.

The (a) modified starch is based on a base starch. Base starch, as used herein, is intended to include all starches derived from any native source, any of which may be suitable for use herein. A native starch, as used herein, is one as it is found in nature. Also suitable are starches derived from a plant obtained by standard breeding techniques including crossbreeding, translocation, inversion, transformation or any other method of gene or chromosome engineering to include variations thereof. In addition, starches derived from a plant grown from artificial mutations and variations of the above generic starches, which may be produced by known standard methods of mutation breeding, are also suitable herein.

Typical sources for the starches are cereals, tubers, roots, legumes and fruits. The native source can be waxy varieties of corn (maize), pea, potato, sweet potato, banana, barley, wheat, rice, oat, sago, amaranth, tapioca (cassava), arrowroot, canna, and sorghum, as well as low and high amylose varieties thereof. As used herein, the term "low amylose" starch is intended to include a starch containing no more than about 10%, particularly no more than 5%, and more particularly no more than 2% amylose by weight. As used herein, the term "high amylose" starch is intended to include a starch containing at least about 50%, particularly at least about 70%, and more particularly at least about 80% amylose by weight. High amylose starches may be preferable.

The (a) modified starch may be pre-gelatinized. Pre-gelatinization and techniques for achieving pre-gelatinization are known in the art and disclosed for example in U.S. Pat. Nos. 4,465,702, 5,037,929, 5,131,953, and 5,149,799. Also see, Chapter XXII-"Production and Use of Pregelatinized Starch", Starch: Chemistry and Technology, Vol. III-Industrial Aspects, R. L. Whistler and E. F. Paschall, Editors, Academic Press, New York 1967. The term pre-gelatinized is intended to mean swollen starch particles, which have lost their birefringence and/or maltese crosses in polarized light. Such pre-gelatinized starch derivatives are substantially soluble in cold water without cooking. In this context "soluble" does not necessarily mean the formation of a true molecular solution, but may also mean a colloidal dispersion. In one embodiment, the starch is completely pre-gelatinized.

The pre-gelatinized modified starch is easily and quickly soluble even in cold water.

Pre-gelatinization may be achieved by methods which include, without limitation, drum drying, extrusion and spray drying. In one embodiment, extrusion is used for the simultaneous cooking and drying of the starch (see for example U.S. Pat. No. 3,137,592). This process makes use of the physical processing of a starch/water mixture at elevated temperatures and pressures which brings about the gelatinization of the starch, followed by expansion after leaving the nozzle with sudden evaporation of the water.

In one embodiment, pre-gelatinization is completed to provide good solubility and eliminate undissolved particles, which may give rise to an unpleasant, sandy feel in the composition.

In one embodiment, the starch has a majority of intact starch granules. Aqueous dispersions of pre-gelatinized starch derivatives having a largely intact granular structure typically have a more uniform smooth texture than aqueous dispersions of starches without a granular structure, which may have a slightly gritty feel. In the case of pre-gelatinized starches with an intact granular structure, the native internal structure of the hydrogen bonds is destroyed, but the external shape or form is maintained.

The (a) modified starch may be crosslinked. Crosslinking of the starch chains can be achieved by suitable crosslinking agents, that is, bifunctional compounds. In one embodiment, the crosslinking method used is phosphorylation, in which the starch is reacted with phosphorous oxychloride, phosphorous pentoxide, and/or sodium trimetaphosphate. Two starch chains are crosslinked by an anionic P-O group. The anionic character of the crosslinking sites assists the emulsion-stabilizing action of the starch to be used according to the present invention. In another embodiment, the crosslinking method is by means of C₄-C₁₈ alkane or alkene dicarboxylic acids which include without limitation C₄-C₈ alkane dicarboxylic acids, exemplified by adipic acid. The alkane or alkene dicarboxylic acid links two starch chains via ester bonds. It can be in straight or branched chain form. The derivatives may be obtained, for example, by reacting starch with the mixed anhydrides of dicarboxylic acid and acetic acid. In one embodiment, less than 0.1 weight percent based on the dry starch crosslinking agent is used. In another embodiment, about 0.06 to 0.1 weight percent based on the dry starch crosslinking agent is used.

It is preferable that the (a) modified starch be hydrophobic. It is more preferable that the surface of the (a) modified starch be hydrophobic.

The modification to make starch hydrophobic may be performed by grafting hydrophobic functional groups such as C₁₋₆ acyl (acetyl), C₁₋₆ hydroxyalkyl (hydroxyethyl or hydroxypropyl), carboxymethyl or octenylsuccinic group.

The alkyl moiety of the functional group may have 1 to 6 carbon atoms, preferably 2 to 5 carbon atoms, and more preferably 3 or 4 carbon atoms.

It is preferable that the (a) modified starch be hydroxylalkyl-modified starch.

The position of the hydroxyl group, which is bound to the starch backbone via an alkyl group such as 2 to 6 carbon atoms in the alkyl group, is not critical and can be in the alpha to omega position. In one suitable embodiment, the degree of substitution of the hydroxyalkylation is about 0.08 to 0.3. The degree of substitution is the average number of substituted OH groups of the starch molecule per anhydroglucose unit. The hydroxyalkylation of a starch can be brought about by reacting a native starch with alkylene oxides with the appropriate number of carbon atoms, including without limitation hydroxypropylation by reaction of the starch with propylene oxide. The hydroxyalkyl-modified starch can also contain more than one hydroxyl group per alkyl group.

The hydroxyalkyl-modified starch may be selected from the group consisting of hydroxyethyl starch, hydroxypropyl starch, hydroxyethyl starch phosphate, hydroxypropyl starch phosphate, and a mixture thereof.

The processes for preparing the hydroxyalkyl-modified starch may be conducted in any order. However, one skilled in the art would understand the advantages of certain orders. For example, hydroxypropylation would typically be conducted before crosslinking, if the starch is crosslinked, with phosphorous oxychloride as the typical hydroxypropylation process would destroy some of the crosslinking achieved.

Examples of the hydroxyalkyl-modified starch preferably used in the present invention may include the following:
Hydroxypropyl starch phosphate (pre-gelatinized, corn starch) marketed by Akzo Nobel as Structure ZEA and XL; and
Corn starch modified (hydroxypropylated, pre-gelatinized, high amylose) marketed by Akzo Nobel, as AMAZE.

The amount of the (a) modified starch in the composition according to the present invention may be 0.01% by weight or more, preferably 0.1% by weight or more, more preferably 0.5% by weight or more, and even more preferably 1% by weight or more, relative to the total weight of the composition.

On the other hand, the amount of the (a) modified starch in the composition according to the present invention may be 15% by weight or less, preferably 10% by weight or less, more preferably 5% by weight or less, and even more preferably 3% by weight or less, relative to the total weight of the composition.

The amount of the (a) modified starch in the composition according to the present invention may range from 0.01% to 15% by weight, preferably from 0.1% to 10% by weight, more preferably from 0.5% to 5% by weight, and even more preferably from 1% to 3% by weight, relative to the total weight of the composition.

### (C₁₃₋C₁₅ Fatty Acid)

The composition according to the present invention comprises (b) at least one C₁₃-C₁₅ fatty acid. A single type of C₁₃-C₁₅ fatty acid, or two or more different types of C₁₃-C₁₅ fatty acids may be used in combination.

It is preferable that the (b) C₁₃-C₁₅ fatty acid is saturated. The saturated C₁₃₋C₁₅ fatty acid can be selected from the group consisting of tridecylic acid (tridecanoic acid), myristic acid (tetradecanoic acid) and pentadecylic acid (pentadecanoic acid).

It is possible that the (b) C₁₃₋C₁₅ fatty acid is unsaturated. The unsaturated C₁₃-C₁₅ fatty acid can be selected from the group consisting of tridecenoic acid, myristoleic acid (tetradecenoic acid) and pentadecenoic acid.

It is more preferable that the (b) C₁₃₋C₁₅ fatty acid be myristic acid.

The amount of the (b) C₁₃-C₁₅ fatty acid in the composition according to the present invention may be 1% by weight or more, preferably 2% by weight or more, more preferably 3% by weight or more, and even more preferably 4% by weight or more, relative to the total weight of the composition.

On the other hand, the amount of the (b) C₁₃-C₁₅ fatty acid in the composition according to the present invention may be 20% by weight or less, preferably 15% by weight or less, more preferably 10% by weight or less, and even more preferably 8% by weight or less; relative to the total weight of the composition.

The amount of the (b) C₁₃-C₁₅ fatty acid in the composition according to the present invention may range from 1% to 20% by weight, preferably from 2% to 15% by weight, more preferably from 3% to 10% by weight, and even more preferably from 4% to 8% by weight, relative to the total weight of the composition.

The weight ratio of the amount (weight) of the (b) C₁₃-C₁₅ fatty acid/the amount (weight) of the (a) modified starch may be 1.0 or more, preferably 2.0 or more, more preferably 3.0 or more, and even more preferably 4.0 or more.

The (a) modified starch and the (b) C₁₃₋C₁₅ fatty acid may form a complex.

### (Clay)

The composition according to the present invention comprises (c) at least one clay. A single type of clay may be used, or two or more different types of clays may be used in combination.

The term "clay" refers to a naturally occurring material composed primarily of fine-grained minerals, which is generally plastic at an appropriate water content and will harden when dried or fired. Although clay usually contains phyllosilicates, it may contain other materials that impart plasticity and harden when dried or fired. Associated phases in clay may include materials that do not impart plasticity and organic matter. A common definition is that in the Penguin Dictionary of Science, namely "finely divided rock materials whose component minerals are various silicates, mainly of magnesium or aluminium". Clay comprises Kaolinite (typically defined as [Si₄]Al₄O₁₀(OH)₈.nH₂O (n=0 or 4)), Illite (typically defined as Mₓ[Si_{6.8}Al_{1.2}]Al₃Fe_{.025}Mg_{.75}O₂₀ (OH)₄), Vermiculite (typically defined as .Mₓ[Si₇Al]AlFe_{.05}Mg_{0.5}O₂₀ (OH)₄), Smectite (typically defined as Mₓ[Si₈]Al_{3.2}Fe_{0.2}Mg_{0.6}O₂₀ (OH)₄, Chlorite (typically defined as (Al(OH)_{2.55})₄[Si_{6.8}AlO_{1.2}}Al_{3.4}Mg_{0.6})₂₀(OH)₄), and Phyllosilicate minerals or talc (typically defined as Mg₃Si₄O₁₀(OH)₂).

Another definition, frequently used by chemists is "a naturally occurring sediment or sedimentary rock composed of one or more minerals and accessory compounds, the whole usually being rich in hydrated aluminum silicate, iron or magnesium, hydrated alumina, or iron oxide, predominating in particles of colloidal or near-colloidal size, and commonly developing plasticity when sufficiently pulverized and wetted" (see Kirk-Othmer, Encyclopaedia of Chemical Technology, Volume 5, page 544, 2nd edition, John Wiley and Sons, Inc., New York, N.Y. 1964). Example of clays are given in the book "Clay mineralogy, S. Caillere, S. Henin, M. Rautureau, 2nd edition 1982, Masson". Clays may be of natural or synthetic origin.

Hydrophilic clay includes smectites such as saponites, hectorites, montmorillonites, bentonites, beidellite. Hydrophilic clay includes synthetic hectorites (also called laponites) such as the products sold by the company under the name Laporte Laponite XLG, Laponite RD, Laponite RDS (these products are sodium silicates and magnesium silicates in particular sodium, lithium and magnesium) bentonites such as the product sold under the name Bentone^{®} HC Rheox, magnesium silicates and aluminum products such as hydrated products sold by Vanderbilt Company as ultra Veegum^{®}, Veegum^{®} HS, Veegum^{®} DGT, or calcium silicates, particularly the synthetic form sold by the company under the name Micro-Cel^{®} C.

Fuller's earth consists chiefly of hydrated aluminum silicates that contain metal ions such as magnesium, sodium, and calcium within their structure. Montmorillonite is the principal clay mineral in fuller's earth, but it may contain other minerals such as kaolinite, attapulgite, and palygorskite among other components.

Lipophilic clay means clay swellable in a lipophilic medium, the clay swells and forms a colloidal dispersion. Lipophilic clays include modified clays such as the modified magnesium silicate (Bentone gel VS38 from Rheox) hectorites modified with an ammonium chloride fatty acid C10 to C22, such as hectorite modified with ammonium chloride distearyldimethylammonium (CTFA name: Disteardimonium hectorite) sold under the name "Bentone 38 CE" by Rheox or Bentone^{®} 38V by ELEMENTIS.

The origin of such clay can be natural or synthetic mineral clay such as hectorite, bentonite, and quaternized derivatives thereof, for example which are obtained by reacting the minerals with a quaternary ammonium compound, such as stearalkonium bentonite, hectorites, quaternized hectorites such as Quaternium-18 hectorite, carbonates such as propylene carbonate, bentones, and the like.

The non-limiting of examples of clay which can be used in the present invention are Fuller's earth, Pinatubo volcanic ash mud from Philippines, Aleppo clay from Syria, Pulau tiga volcano mud from Malasiya, Nha Trang mud from Vietnam, White Kaolinite from Korea, Yellow Loess from Korea, Jeju volcanic clay from Korea, Guanziling mud form Taiwan, Wudalianchi volcanic mud from China, Black mud of Yuncheng salt lake from China, mineral mud from Tantou village in China, China clay (Kaolin), Maifan stone from China, Beppu onsen Fango from Japan, Kucha from Japan, Tanakura clay from Japan, Cambrian blue clay from Russia, Blue Lagoon mud from Iceland, Saki lake mud from Ukraine, Karlovy Vary moor mud from Czech Republic, Heviz Georgikon moor mud from Hungry, Alpine moor mud from Austria, Bad Wilsnack mud from Germany, Bavarian mineral slat mountain mud from Germany, Freiburg volcanic ash from Germany, Santorini mud from Greece, Mar Menor mud from Spian, Ischian volcanic mud from Italy, Euganean thermal mud from Italy, Yellow clay-Illite from France, French Green Clay--Montmorrillonite, Calistoga mud from USA, Sacred clay and ormalite from USA, Redmond clay from USA, Arctic mineral mud from Canada, Tulum Mayan clay from Mexico, Glacial clay from Canada, Amazonian white clay from Brazil, El Chillante volcanic thermal mud from Argentina, African healing clay, Australian olive green clay.

It is preferable that the (c) clay be selected from the group consisting of hectorite, kaolin, talc, and a mixture thereof.

The amount of the (c) clay in the composition according to the present invention may be 1% by weight or more, preferably 2% by weight or more, and more preferably 3% by weight or more, relative to the total weight of the composition.

On the other hand, the amount of the (c) clay in the composition according to the present invention may be 40% by weight or less, preferably 35% by weight or less, and more preferably 30% by weight or less, relative to the total weight of the composition.

The amount of the (c) clay in the composition according to the present invention may range from 1% to 40% by weight, preferably from 2% to 35% by weight, and more preferably from 3% to 30% by weight, relative to the total weight of the composition.

The (c) clay may be coated with the (a) modified starch and the (b) C₁₃-C₁₅ fatty acid, preferably a complex formed by the (a) modified starch and the (b) C₁₃-C₁₅ fatty acid, and more preferably a complex formed by hydrophobic modified starch and myristic acid.

### (Amphoteric Surfactant)

The composition according to the present invention comprises (d) at least one amphoteric surfactant. Two or more amphoteric surfactants may be used. Thus, a single type of surfactant or a combination of different types of amphoteric surfactants may be used.

The amphoteric or zwitterionic surfactants can be, for example (non-limiting list), amine derivatives such as aliphatic secondary or tertiary amine, and optionally quaternized amine derivatives, in which the aliphatic radical is a linear or branched chain including 8 to 22 carbon atoms and containing at least one water-solubilizing anionic group (for example, carboxylate, sulphonate, sulphate, phosphate or phosphonate).

The amphoteric surfactant may preferably be selected from the group consisting of betaines and amidoaminecarboxylated derivatives.

It is preferable that the amphoteric surfactant be selected from betaine-type surfactants.

The betaine-type amphoteric surfactant is preferably selected from the group consisting of alkylbetaines, alkylamidoalkylbetaines, sulfobetaines, alkylsulfobetaines, phosphobetaines, alkylphosphobetaines, and alkylamidoalkylsulfobetaines, in particular, (C₈-C₂₄)alkylbetaines, (C₈-C₂₄)alkylamido(C₁-C₈)alkylbetaines, sulfobetaines, (C₁-C₈)alkylsulfobetaines, phosphobetaines, (C₁-C₈)alkylphosphobetaines, and (C₈-C₂₄)alkylamido(C₁-C₈)alkylsulfobetaines. In one embodiment, the amphoteric surfactants of betaine type are chosen from (C₈-C₂₄)alkylbetaines, (C₈-C₂₄)alkylamido(C₁-C₈)alkylsulfobetaines, sulfobetaines, (C₁-C₈)alkylsulfobetaines and phosphobetaines.

Non-limiting examples that may be mentioned include the compounds classified in the CTFA International Cosmetic Ingredient Dictionary & Handbook, 15th Edition, 2014, under the names cocobetaine, laurylbetaine, cetylbetaine, coco/oleamidopropylbetaine, cocamidopropylbetaine, palmitamidopropylbetaine, stearamidopropylbetaine, cocamidoethylbetaine, cocamidopropylhydroxysultaine, oleamidopropylhydroxysultaine, cocohydroxysultaine, laurylhydroxysultaine, and cocosultaine, alone or as mixtures.

The betaine-type amphoteric surfactant (betaines) is preferably an alkylbetaine an alkylsulfobetaine, and an alkylamidoalkylbetaine, in particular cocobetaine, sulfopropylbetaine, and cocamidopropylbetaine.

Among the amidoaminecarboxylated derivatives, mention may be made of the products sold under the name Miranol, as described in U.S. Pat. Nos. 2,528,378 and 2,781,354 and classified in the CTFA dictionary, 3rd edition, 1982 under the names Amphocarboxyglycinates and Amphocarboxypropionates, with the respective structures:

R₁-CONHCH₂CH₂-N⁺(R₂)(R₃)(CH₂COO⁻) M⁺ X⁻ (B1)

in which:
R₁ denotes an alkyl radical of an acid R₁-COOH present in hydrolysed coconut oil, a heptyl, nonyl or undecyl radical,
R₂ denotes a beta-hydroxyethyl group,
R₃ denotes a carboxymethyl group,
M⁺ denotes a cationic ion derived from alkaline metals such as sodium; ammonium ion; or an ion derived from an organic amine;
X⁻ denotes an organic or inorganic anionic ion such as halides, acetates, phosphates, nitrates, alkyl(C₁-C₄)sulfates, alkyl(C₁-C₄)- or alkyl(C₁-C₄)aryl-sulfonates, particularly methylsulfate and ethylsulfate; or M⁺ and X⁻ are not present;

   R₁'-CONHCH₂CH₂-N(B)(C) (B2)

   in which:
   R₁' denotes an alkyl radical of an acid R₁'-COOH present in coconut oil or in hydrolysed linseed oil, an alkyl radical, such as a C₇, C₉, C₁₁ or C₁₃ alkyl radical, a C₁₇ alkyl radical and its iso-form, or an unsaturated C₁₇ radical,
   B represents -CH₂CH₂OX',
   C represents -(CH₂)_{z}-Y', with z=1 or 2,
   X' denotes a -CH₂-COOH group, -CH₂-COOZ', -CH₂CH₂-COOH, -CH₂CH₂-COOZ' or a hydrogen atom, and
   Y' denotes -COOH, -COOZ', -CH₂-CHOH-SO₃Z', -CH₂-CHOH-SO₃H radical or a -CH₂-CH(OH)-SO₃-Z' radical,
   wherein Z' represents an ion of an alkaline or alkaline earth metal such as sodium, an ion derived from an organic amine or an ammonium ion;
      and

      R_{a"'}-NH-CH(Y")-(CH₂)ₙ-C(O)-NH-(CH₂)_{n'}-N(Rd)(Re) (B'2)

      in which:
      Y" denotes -C(O)OH, -C(O)OZ", -CH₂-CH(OH)-SO₃H or -CH₂-CH(OH)-SO₃-Z", wherein Z" denotes a cationic ion derived from alkaline metal or alkaline-earth metals such as sodium, an ion derived from organic amine or an ammonium ion;
      Rd and Re denote a C₁-C₄ alkyl or C₁-C₄ hydroxyalkyl radical;
      R_{a"} denotes a C₁₀-C₃₀ group alkyl or alkenyl group from an acid, and
      n and n' independently denote an integer from 1 to 3.

It is preferable that the amphoteric surfactant with formula B1 and B2 be selected from (C₈-C₂₄)-alkyl amphomonoacetates, (C₈-C₂₄)alkyl amphodiacetates, (C₈-C₂₄)alkyl amphomonopropionates, and (C₈-C₂₄)alkyl amphodipropionates

These compounds are classified in the CTFA dictionary, 5th edition, 1993, under the names Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphopropionate, Disodium Caprylamphodipropionate, Disodium Caprylamphodipropionate, Lauroamphodipropionic acid and Cocoamphodipropionic acid.

By way of example, mention may be made of the cocoamphodiacetate sold under the trade name Miranol^{®} C2M concentrate by the company Rhodia Chimie.

Among compounds of formula (B'2), mention may be made of sodium diethylaminopropyl cocoaspartamide (CTFA) marketed by CHIMEX under the denomination CHIMEXANE HB.

The amphoteric surfactant may be selected from N-acylamino acids such as N-alkyl aminoacetates and disodium cocoamphodi acetate, and amine oxides such as stearamine oxide and lauramine oxide.

It is also preferable that the amphoteric surfactant be selected from amine oxides such as stearamine oxide and lauramine oxide.

The amount of the (d) amphoteric surfactant in the composition according to the present invention may be 1% by weight or more, preferably 2% by weight or more, and more preferably 3% by weight or more, relative to the total weight of the composition.

On the other hand, the amount of the (d) amphoteric surfactant in the composition according to the present invention may be 20% by weight or less, preferably 15% by weight or less, and more preferably 10% by weight or less, relative to the total weight of the composition.

The amount of the (d) amphoteric surfactant in the composition according to the present invention may range from 1% to 20% by weight, preferably from 2% to 15% by weight, and more preferably from 3% to 10% by weight, relative to the total weight of the composition.

According to the present invention, the weight ratio of the amount of the (d) amphoteric surfactant/the amount of the (b) C₁₃₋C₁₅ fatty acid is 0.5 or more, preferably 0.6 or more, and more preferably 0.7 or more.

It may be preferable that the weight ratio of the amount of the (d) amphoteric surfactant/the amount of the (b) C₁₃-C₁₅ fatty acid is 10 or less, more preferably 5 or less, and more preferably 3 or less.

Since the (d) amphoteric surfactant has a large number of cationic moieties, the use of the (d) amphoteric surfactant in a weight of 50% by weight or more (preferably 60% by weight or more, and more preferably 70% by weight or more) relative to the weight of the (b) C₁₃₋C₁₅ fatty acid (with a monovalent anionic moiety) can be sufficient.

### (Water)

The composition according to the present invention may further comprise (e) water.

The (e) water can form a carrier of the ingredients (a) to (d) in the composition according to the present invention.

The amount of the (e) water may be 30% by weight or more, preferably 35% by weight or more, and more preferably 40% by weight or more, relative to the total weight of the composition.

The amount of the (e) water may be 70% by weight or less, preferably 65% by weight or less, and more preferably 60% by weight or less, relative to the total weight of the composition.

The amount of the (e) water may be from 30% to 70% by weight, preferably from 35% to 65% by weight, and more preferably from 40% to 60% by weight, relative to the total weight of the composition.

### (pH)

The pH of the composition according to the present invention may be adjusted to the desired value using acidifying or basifying agents commonly used in cosmetic products.

The composition according to the present invention may be acidic. For example, the pH of the composition according to the present invention may be less than 7.0, more preferably less than 6.5, and even more preferably less than 6.0.

Alternatively, the composition according to the present invention may be basic. For example, the pH of the composition according to the present invention may be more than 7.0, more preferably more than 7.5, and even more preferably more than 8.0.

Among the acidifying agents, mention may be made, by way of example, of mineral or organic acids such as hydrochloric acid, ortho-phosphoric acid, sulfuric acid, carboxylic acids such as acetic acid, tartaric acid, citric acid, and lactic acid, and sulfonic acids.

Among the basifying agents, mention may be made, by way of example, of ammonium hydroxide, alkali metal carbonates, alkanolamines such as mono-, di- and triethanolamines and also their derivatives, alkali metal hydroxides such as sodium or potassium hydroxide and compounds of the formula below: 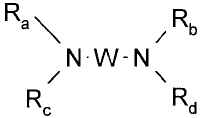 wherein
W denotes an alkylene such as propylene optionally substituted by a hydroxyl or a C₁-C₄ alkyl radical, and Rₐ, R_{b}, R_{c} and R_{d} independently denote a hydrogen atom, an alkyl radical or a C₁-C₄ hydroxyalkyl radical, which may be exemplified by 1,3-propanediamine and derivatives thereof.

The acidifying or basifying agent may be used in an amount of 20% by weight or less, preferably 15% by weight or less, and more preferably 10% by weight or less, relative to the total weight of the composition.

The acidifying or basifying agent may be used in an amount of 0.001% by weight or more, preferably 0.01% by weight or more, and more preferably 0.1% by weight or more, relative to the total weight of the composition.

The acidifying or basifying agent may be used in an amount ranging from 0.001% to 20% by weight, preferably from 0.01% to 15% by weight, and more preferably from 0.1% to 10% by weight, relative to the total weight of the composition.

### (Additional Optional Ingredients)

The composition according to the present invention may also include any other optional or additional ingredient(s).

The other optional ingredient(s) may be selected from the group consisting of anionic, cationic, nonionic, or amphoteric polymers; fillers; pigments; inorganic and organic UV filters; peptides and derivatives thereof; protein hydrolyzates; swelling agents and penetrating agents; agents for combating hair loss; anti-dandruff agents; suspending agents; sequestering agents; opacifying agents; dyes; vitamins or provitamins; fragrances; preserving agents, stabilizers; and mixtures thereof.

The composition according to the present invention may include one or several cosmetically acceptable organic solvents, which may be alcohols: in particular monovalent alcohols such as ethyl alcohol, isopropyl alcohol, benzyl alcohol, and phenylethyl alcohol; diols such as ethylene glycol, propylene glycol, and butylene glycol; other polyols such as glycerol, sugar, and βs; and ethers such as ethylene glycol monomethyl, monoethyl, and monobutyl ethers, propylene glycol monomethyl, monoethyl, and monobutyl ethers, and butylene glycol monomethyl, monoethyl, and monobutyl ethers.

The organic solvent(s) may be present in a concentration of from 0.01% to 25% by weight, preferably from 0.1% to 20% by weight, and more preferably from 1% to 15% by weight, relative to the total weight of the composition.

### [Form]

It is preferable that the composition according to the present invention be in the form of a liquid at 25°C and under atmospheric pressure (760 mmHg).

The composition according to the present invention may be in any form such as a solution, a dispersion, a gel, and a paste.

The composition according to the present invention may be a cosmetic composition. Thus, the cosmetic composition according to the present invention may be intended for application onto a keratin substance. Keratin substance here means a material containing keratin as a main constituent element, and examples thereof include the skin, scalp, nails, lips, hair, and the like. Thus, it is preferable that the cosmetic composition according to the present invention be. used for a cosmetic process for the keratin substance, in particular skin.

The composition according to the present invention may preferably be a rinse-off composition. The rinse-off composition can be removed from a keratin substance such as skin, preferably with water.

The composition according to the present invention may preferably be a cleansing composition. The cleansing composition can remove sebum and/or makeup on a keratin substance such as skin from the keratin substance.

The composition according to the present invention may more preferably be a rinse-off cleansing composition. The rinse-off cleansing composition can remove sebum and/or makeup on a keratin substance such as skin, and can be removed from the keratin substance, preferably with water.

The composition according to the present invention can be prepared by mixing the above-described essential and optional ingredients in a conventional manner.

### [Process]

The present invention also relates to a cosmetic process for a keratin substrate, such as skin, comprising: applying to the keratin substrate the composition according to the present invention.

It is preferable that the cosmetic process according to the present invention further comprise rinsing off the composition according to the present invention which has been applied onto the keratin substance from the keratin substance.

It is possible that the composition according to the present invention be left on the keratin substance for a certain period of time such as some minutes after being applied onto the keratin substance.

The cosmetic process here means a non-therapeutic cosmetic method, preferably for cleansing the keratin substance such as skin, and more preferably for cleansing sebum and/or makeup on the keratin substance.

### [Use]

The present invention also relates to a use of a combination of:
(a) at least one modified starch;
(b) at least one C₁₃-C₁₅ fatty acid; and
(d) at least one amphoteric surfactant,
wherein
the weight ratio of the amount of the (d) amphoteric surfactant/the amount of the (b) C₁₃-C₁₅ fatty acid is 0.5 or more, preferably 0.6 or more, and more preferably 0.7 or more,
in order to increase the deposition on a keratin substance, such as skin, of (c) at least one clay.

In other words, the combination of the ingredients (a), (b) and (d) can enhance the deposition on the keratin substance such as skin of the ingredient (c).

The above explanations for the ingredients (a) to (d) for the composition according to the present invention can also apply to the above ingredients (a) to (d) for the use according to the present invention.

The (a) modified starch and the (b) C₁₃₋C₁₅ fatty acid may form a complex, and preferably the (c) clay is coated with the complex.

The hydrophobicity of the (c) clay can be enhanced by the (a) modified starch and the (b) C₁₃-C₁₅ fatty acid, preferably a complex formed by the (a) modified starch and the (b) C₁₃-C₁₅ fatty acid, and more preferably a complex formed by hydrophobic modified starch and myristic acid. Therefore, the (c) clay can deposit more on a keratin substance such as skin, due to hydrophobic-hydrophobic interaction between the (c) clay and the keratin substance. This can result in the increase in the amount of the deposition of the (c) clay on the keratin substance.

Furthermore, surprisingly, the additional use of the (d) amphoteric surfactant can further enhance the deposition of the (c) clay on a keratin substance such as skin.

Without being bound by theory, it is believed that the cationic moieties of the (d) amphoteric surfactant can reduce the negative charges of a possible complex of the (a) modified starch and the (b) C₁₃-C₁₅ fatty acid on the (c) clay, which could be attributed to the (b) C₁₃₋C₁₅ fatty acid, and therefore, the (c) clay with the possible complex having reduced negative charges can be relatively easily absorbed on the keratin substance, such as skin, which inherently has negative charges.

Since the use according to the present invention can increase the amount of the deposition of clay on a keratin substance, the use according to the present invention can provide the keratin substance with effects such as immediate and long-lasting oil control effects. Also, the increased amount of the deposition of clay on a keratin substance such as skin can provide a smooth feeling to touch for a long period of time.

The above combination can be used in a composition, preferably a cosmetic composition, and more preferably a cleansing cosmetic composition, which may include the optional ingredients such as water and at least one additional optional ingredients such as those explained above, in particular at least one acidifying agent and/or at least one basifying agent.

The above composition may be acidic. For example, the pH of the above composition may be less than 7.0, more preferably less than 6.5, and even more preferably less than 6.0. Alternatively, the above composition may be basic. For example, the pH of the above composition may be more than 7.0, more preferably more than 7.5, and even more preferably more than 8.0. c

The use according to the present invention may be rephrased as a process for increasing or enhancing the deposition on a keratin substance, such as skin, of (c) at least one clay, comprising applying a combination of (a) modified starch, (b) C₁₃₋C₁₅ fatty acid, and (d) amphoteric surfactant, wherein the weight ratio of the amount of the (d) amphoteric surfactant/the amount of the (b) C₁₃-C₁₅ fatty acid is 0.5 or more, preferably 0.6 or more, and more preferably 0.7 or more, with the (c) clay onto the keratin substance. This process can increase the amount of the deposition of the (c) clay on the keratin substance.

### EXAMPLES

The present invention will be described in a more detailed manner by way of examples. However, these examples should not be construed as limiting the scope of the present invention. The examples below are presented as non-limiting illustrations in the field of the present invention.

### Examples 1 and Comparative Example 1

### [Preparations]

Each of the treatment compositions for hair according to Examples 1 (Ex. 1) and Comparative Example 1 (Comp. Ex. 1) was prepared by mixing the ingredients shown in Table 1. The numerical values for the amounts of the ingredients are all based on "% by weight" as active materials.

**Table 1**

| | Ex. 1 | Comp. Ex. 1 |
|---|---|---|
| Corn Starch Modified | 1.00 | 1.00 |
| Lauric Acid | 5.63 | 5.33 |
| Myristic Acid | 4.50 | 8.44 |
| Palmitic Acid | - | 6.66 |
| Stearic Acid | 1.25 | 4.00 |
| Kaolin | 10.00 | 10.00 |
| Coco-Betaine | 4.50 | - |
| Potassium Cocoyl Glycinate (and) Potasium Cocoate | - | 10.00 |
| Glycerin | 8.00 | 5.00 |
| Propylene Glycol | 2.00 | - |
| Butylene Glycol | - | 3.00 |
| Caprylyl Glycol | - | 1.00 |
| Glycol Distearate | 1.25 | - |
| Glyceryl Stearate SE | - | 1.33 |
| Acrylates Copolymer | 5.50 | - |
| Phenoxyethanol | 0.70 | 0.50 |
| Tetrasodium Glutamate Diacetate | 0.10 | - |
| Tetrasodium EDTA | - | 0.50 |
| Potassium Hydroxide | 3.34 | 9.70 |
| Water | qsp 100 | qsp 100 |

### [Evaluations]

The shine control efficacy provided by each of the compositions according to Example 1 and Comparative Example 1 was evaluated by instrumental methodology of Lightcam (Newtone Technologies, France) on 19 healthy female subjects with oily skin.

The conditions of the instrument measurement were 21+/-1 °C and 45+/-5%RH.

500 mg of each of the compositions according to Example 1 and Comparative Example 1 was applied onto half of the face of subject. Immediately after the application (T0) and 4 hours after the application (T4h), the gross on the forehead was measured at the applied half face and the unapplied half face. The difference in gloss between the gloss at the applied half face and that at the unapplied half face was averaged to determine a mean score of gloss control.

The results are shown in Fig. 1.

As shown in Fig. 1 (A), the composition according to Example 1 can provide better shine control efficacy than the composition according to Comparative Example 1, even just after the application thereof. Thus, the composition according to Example 1 can provide advantageous immediate shine controlling effects.

As shown in Fig. 1 (B), the compositions according to Example 1 can provide better shine control efficacy than the composition according to Comparative Example 1, even 4 hours after the application thereof. Thus, the composition according to Example 1 can provide advantageous long-lasting shine controlling effects.

### Examples 1-4 and Comparative Examples 2 and 3

### [Preparations]

Each of the treatment compositions for hair according to Examples 1-4 (Ex. 1-4) and Comparative Examples 2 and 3 (Comp. Ex. 2-3) was prepared by mixing the ingredients shown in Table 2. The numerical values for the amounts of the ingredients are all based on "% by weight" as active materials.

**Table 2**

| | Ex. 1 | Ex. 2 | Ex. 3 | Ex. 4 | Comp. Ex. 2 | Comp. Ex. 3 |
|---|---|---|---|---|---|---|
| Corn Starch Modified | 1.00 | 1.00 | 1.00 | 1.00 | 1.00 | 1.00 |
| Lauric Acid | 6.75 | 6.75 | 6.75 | 6.75 | 6.75 | 6.75 |
| Myristic Acid | 5.40 | 5.40 | 5.40 | 5.40 | 5.40 | 5.40 |
| Stearic Acid | 1.50 | 1.50 | 1.50 | 1.50 | 1.50 | 1.50 |
| Kaolin | 10.00 | 10.00 | 10.00 | 10.00 | 10.00 | 10.00 |
| Coco-Betaine | 4.50 | - | - | - | - | - |
| Lauramine Oxide | - | 4.5 | - | - | - | - |
| Sulfopropyl Betaine | - | - | 4.5 | - | - | - |
| Cocamidopropyl Betaine | - | - | - | 4.5 | - | - |
| Coco-Glucoside | - | - | - | - | 4.5 | - |
| Sodium Laureth Sulfate | - | - | - | - | - | 4.5 |
| Glycerin | 8.00 | 8.00 | 8.00 | 8.00 | 8.00 | 8.00 |
| Propylene Glycol | 2.00 | 2.00 | 2.00 | 2.00 | 2.00 | 2.00 |
| Glycol Distearate | 1.50 | 1.50 | 1.50 | 1.50 | 1.50 | 1.50 |
| Acrylates Copolymer | 5.50 | 5.50 | 5.50 | 5.50 | 5.50 | 5.50 |
| Phenoxyethanol | 0.70 | 0.70 | 0.70 | 0.70 | 0.70 | 0.70 |
| Tetrasodium Glutamate Diacetate | 0.10 | 0.10 | 0.10 | 0.10 | 0.10 | 0.10 |
| Potassium Hydroxide | 3.73 | 3.73 | 3.73 | 3.73 | 3.73 | 3.73 |
| Water | qsp 100 | qsp 100 | qsp 100 | qsp 100 | qsp 100 | qsp 100 |
| Deposition Feeling | 4.83 | 4.50 | 4.87 | 5.00 | 3.33 | 3.16 |
| Long-Lasting Oil Control | 4.67 | 4.33 | 4.67 | 5.00 | 3.67 | 3.50 |

### [Evaluations]

### (Sensory Evaluations)

The compositions according to Examples 1-4 and Comparative Examples 2 and 3 were evaluated with respect to deposition feeling and long-lasting oil control by 3 monitors. Each of the compositions according to Examples 1-4 and Comparative Examples 2 and 3 was applied on the monitors' faces by the monitors themselves. Each composition was rinsed off. The monitors evaluated deposition feeling as a level of quantity of clay remaining on the surface of the skin after rising, by scoring in accordance with the following criteria. The average score is shown in Table 2.
5: Very Good
4: Good
3: Fair
2: Poor
1: Very Poor

Also, the monitors evaluated long lasting oil control as a level of maintaining no/less oily shine and non-greasy/sticky feeling to touch, 4 hours after rising, by scoring in accordance with the following criteria. The average score is shown in Table 2.
5: Very Good
4: Good
3: Fair
2: Poor
1: Very Poor

It is clear from Table 2 that the compositions according to Examples 1-4, which includes a combination of (a) modified starch, (b) C₁₃₋C₁₅ fatty acid, and (d) amphoteric surfactant, with (c) clay, was able to provide the best results in terms of deposition feeling and long-lasting oil control.

On the other hand, the composition according to Comparative Example 2, which includes a nonionic surfactant (coco-glucoside) instead of the (d) amphoteric surfactant, provided inferior results, as compared to the composition according to Examples 1-4, in terms of deposition feeling and long-lasting oil control.

Also, the composition according to Comparative Example 3, which includes an anionic surfactant (sodium laureth sulfate) instead of the (d) amphoteric surfactant, provided inferior results, as compared to the composition according to Examples 1-4, in terms of deposition feeling and long-lasting oil control.

## Claims

1. A composition, comprising:
(a) at least one modified starch;
(b) at least one C₁₃₋C₁₅ fatty acid;
(c) at least one clay; and
(d) at least one amphoteric surfactant,
wherein
the weight ratio of the amount of the (d) amphoteric surfactant/the amount of the (b) C₁₃₋C₁₅ fatty acid is 0.5 or more, preferably 0.6 or more, and more preferably 0.7 or more.

2. The composition according to Claim 1, wherein the (a) modified starch is hydrophobic, preferably hydroxyalkyl-modified starch, and more preferably selected from the group consisting of hydroxyethyl starch, hydroxypropyl starch, hydroxyethyl starch phosphate, hydroxypropyl starch phosphate, and a mixture thereof.

3. The composition according to Claim 1 or 2, wherein the amount of the (a) modified starch in the composition is from 0.01% to 15% by weight, preferably from 0.1% to 10% by weight, and more preferably from 0.5% to 5% by weight, relative to the total weight of the composition.

4. The composition according to any one of Claims 1 to 3, wherein the (b) C₁₃₋C₁₅ fatty acid is myristic acid.

5. The composition according to any one of Claims 1 to 4, wherein the amount of the (b) C₁₃₋C₁₅ fatty acid in the composition is from 1% to 20% by weight, preferably from 2% to 15% by weight, and more preferably from 3% to 10% by weight, relative to the total weight of the composition.

6. The composition according to any one of Claims 1 to 5, wherein the (c) clay is selected from the group consisting of hectorite, kaolin, talc, and a mixture thereof.

7. The composition according to any one of Claims 1 to 6, wherein the amount of the (c) clay in the composition is from 1% to 40% by weight, preferably from 2% to 35% by weight, and more preferably from 3% to 30% by weight, relative to the total weight of the composition.

8. The composition according to any one of Claims 1 to 7, wherein the (a) modified starch and the (b) C₁₃₋C₁₅ fatty acid form a complex.

9. The composition according to any one of Claims 1 to 8, wherein the (d) amphoteric surfactant is selected from betaines, amine oxides, and mixtures thereof.

10. The composition according to any one of Claims 1 to 9, wherein the amount of the (d) amphoteric surfactant in the composition is from 1% to 20% by weight, preferably from 2% to 15% by weight, and more preferably from 3% to 10% by weight, relative to the total weight of the composition.

11. The composition according to any one of Claims 1 to 10, wherein the composition further comprises (e) water.

12. The composition according to Claim 11, wherein the pH of the composition is more than 7.0, preferably more than 7.5, and more preferably more than 8.0.

13. The composition according to any one of Claims 1 to 12, wherein the composition is a cosmetic composition, preferably a rinse-off composition, and more preferably a rinse-off cleansing composition.

14. A cosmetic process for a keratin substance, such as skin, comprising the step of: applying the composition according to any one of Claims 1 to 13 onto the keratin substance.

15. A use of a combination of:
(a) at least one modified starch;
(b) at least one C₁₃-C₁₅ fatty acid; and
(d) at least one amphoteric surfactant,
wherein
the weight ratio of the amount of the (d) amphoteric surfactant/the amount of the (b) C₁₃₋C₁₅ fatty acid is 0.5 or more, preferably 0.6 or more, and more preferably 0.7 or more,
in order to increase the deposition on a keratin substance, such as skin, of (c) at least one clay.

## Patentansprüche

1. Zusammensetzung, umfassend:
(a) wenigstens eine modifizierte Stärke;
(b) wenigstens eine C₁₃-C₁₅-Fettsäure;
(c) wenigstens einen Ton; und
(d) wenigstens ein amphoteres Tensid,
wobei
das Gewichtsverhältnis der Menge des (d) amphoteren Tensids zur Menge der (b) C₁₃-C₁₅-Fettsäure 0,5 oder mehr, vorzugsweise 0,6 oder mehr und besonders bevorzugt 0,7 oder mehr beträgt.

2. Zusammensetzung nach Anspruch 1, wobei die (a) modifizierte Stärke hydrophob, vorzugsweise mit einem Hydroxyalkyl modifizierte Stärke und besonders bevorzugt aus der Gruppe bestehend aus Hydroxyethylstärke, Hydroxypropylstärke, Hydroxyethylstärkephosphat, Hydroxypropylstärkephosphat und einem Gemisch davon ausgewählt ist.

3. Zusammensetzung nach Anspruch 1 oder 2, wobei die Menge der (a) modifizierten Stärke in der Zusammensetzung 0,01 Gew.-% bis 15 Gew.-%, vorzugsweise 0,1 Gew.-% bis 10 Gew.-% und besonders bevorzugt 0,5 Gew.-% bis 5 Gew.-%, bezogen auf das Gesamtgewicht der Zusammensetzung, beträgt.

4. Zusammensetzung nach einem der Ansprüche 1 bis 3, wobei die (b) C₁₃-C₁₅-Fettsäure Myristinsäure ist.

5. Zusammensetzung nach einem der Ansprüche 1 bis 4, wobei die Menge der (b) C₁₃-C₁₅-Fettsäure in der Zusammensetzung 1 Gew.-% bis 20 Gew.-%, vorzugsweise 2 Gew.-% bis 15 Gew.-% und besonders bevorzugt 3 Gew.-% bis 10 Gew.-%, bezogen auf das Gesamtgewicht der Zusammensetzung, beträgt.

6. Zusammensetzung nach einem der Ansprüche 1 bis 5, wobei der (c) Ton aus der Gruppe bestehend aus Hectorit, Kaolin, Talk und einem Gemisch davon ausgewählt ist.

7. Zusammensetzung nach einem der Ansprüche 1 bis 6, wobei die Menge des (c) Tons in der Zusammensetzung 1 Gew.-% bis 40 Gew.-%, vorzugsweise 2 Gew.-% bis 35 Gew.-% und besonders bevorzugt 3 Gew.-% bis 30 Gew.-%, bezogen auf das Gesamtgewicht der Zusammensetzung, beträgt.

8. Zusammensetzung nach einem der Ansprüche 1 bis 7, wobei die (a) modifizierte Stärke und die (b) C₁₃-C₁₅-Fettsäure einen Komplex bilden.

9. Zusammensetzung nach einem der Ansprüche 1 bis 8, wobei das (d) amphotere Tensid aus Betainen, Aminoxiden und Gemischen davon ausgewählt ist.

10. Zusammensetzung nach einem der Ansprüche 1 bis 9, wobei die Menge des (d) amphoteren Tensids in der Zusammensetzung 1 Gew.-% bis 20 Gew.-%, vorzugsweise 2 Gew.-% bis 15 Gew.-% und besonders bevorzugt 3 Gew.-% bis 10 Gew.-%, bezogen auf das Gesamtgewicht der Zusammensetzung, beträgt.

11. Zusammensetzung nach einem der Ansprüche 1 bis 10, wobei die Zusammensetzung ferner (e) Wasser umfasst.

12. Zusammensetzung nach Anspruch 11, wobei der pH-Wert der Zusammensetzung mehr als 7,0, vorzugsweise mehr als 7,5 und besonders bevorzugt mehr als 8,0 beträgt.

13. Zusammensetzung nach einem der Ansprüche 1 bis 12, wobei die Zusammensetzung eine kosmetische Zusammensetzung, vorzugsweise eine abzuspülende Zusammensetzung und besonders bevorzugt eine abzuspülende Reinigungszusammensetzung ist.

14. Kosmetisches Verfahren für eine Keratinsubstanz, wie etwa Haut, das folgenden Schritt umfasst:
Auftragen der Zusammensetzung nach einem der Ansprüche 1 bis 13 auf die Keratinsubstanz.

15. Verwendung einer Kombination von:
(a) wenigstens einer modifizierten Stärke;
(b) wenigstens einer C₁₃-C₁₅-Fettsäure; und
(d) wenigstens einem amphoteren Tensid,
wobei
das Gewichtsverhältnis der Menge des (d) amphoteren Tensids zur Menge der (b) C₁₃-C₁₅-Fettsäure 0,5 oder mehr, vorzugsweise 0,6 oder mehr und besonders bevorzugt 0,7 oder mehr beträgt,
um die Ablagerung von (c) wenigstens einem Ton auf einer Keratinsubstanz, wie etwa Haut, zu erhöhen.

## Revendications

1. Composition comprenant :
(a) au moins un amidon modifié ;
(b) au moins un acide gras en C₁₃ à C₁₅ ;
(c) au moins une argile ; et
(d) au moins un tensioactif amphotère,
dans laquelle
le rapport pondéral de la quantité du tensioactif amphotère (d)/quantité de l'acide gras en C₁₃ à C₁₅ (b) est supérieur ou égal à 0,5, de préférence supérieur ou égal à 0,6, et plus préférablement supérieur ou égal à 0,7.

2. Composition selon la revendication 1, dans laquelle l'amidon modifié (a) est hydrophobe, de préférence un amidon modifié par un groupe hydroxyalkyle, et plus préférablement choisi dans le groupe constitué par l'hydroxyéthylamidon, l'hydroxypropylamidon, le phosphate d'hydroxyéthylamidon, le phosphate hydroxypropylamidon et un mélange de ceux-ci.

3. Composition selon la revendication 1 ou 2, dans laquelle la quantité de l'amidon modifié (a) dans la composition est de 0,01 % à 15 % en poids, de préférence de 0,1 % à 10 % en poids, et plus préférablement de 0,5 % à 5 % en poids, par rapport au poids total de la composition.

4. Composition selon l'une quelconque des revendications 1 à 3, dans laquelle l'acide gras en C₁₃ à C₁₅ (b) est l'acide myristique.

5. Composition selon l'une quelconque des revendications 1 à 4, dans laquelle la quantité de l'acide gras en C₁₃ à C₁₅ (b) dans la composition est de 1 % à 20 % en poids, de préférence de 2 % à 15 % en poids, et plus préférablement de 3 % à 10 % en poids, par rapport au poids total de la composition.

6. Composition selon l'une quelconque des revendications 1 à 5, dans laquelle l'argile (c) est choisie dans le groupe constitué par l'hectorite, le kaolin, le talc et un mélange de ceux-ci.

7. Composition selon l'une quelconque des revendications 1 à 6, dans laquelle la quantité de l'argile (c) dans la composition est de 1 % à 40 % en poids, de préférence de 2 % à 35 % en poids, et plus préférablement de 3 % à 30 % en poids, par rapport au poids total de la composition.

8. Composition selon l'une quelconque des revendications 1 à 7, dans laquelle l'amidon modifié (a) et l'acide gras en C₁₃ à C₁₅ (b) forment un complexe.

9. Composition selon l'une quelconque des revendications 1 à 8, dans laquelle le tensioactif amphotère (d) est choisi parmi les bétaïnes, les oxydes d'amine et des mélanges de ceux-ci.

10. Composition selon l'une quelconque des revendications 1 à 9, dans laquelle la quantité du tensioactif amphotère (d) dans la composition est de 1 % à 20 % en poids, de préférence de 2 % à 15 % en poids, et plus préférablement de 3 % à 10 % en poids, par rapport au poids total de la composition.

11. Composition selon l'une quelconque des revendications 1 à 10, dans laquelle la composition comprend en outre (e) de l'eau.

12. Composition selon la revendication 11, dans laquelle le pH de la composition est supérieur à 7,0, de préférence supérieur à 7,5, et plus préférablement supérieur à 8,0.

13. Composition selon l'une quelconque des revendications 1 à 12, dans laquelle la composition est une composition cosmétique, de préférence une composition à rincer, et plus préférablement une composition nettoyante à rincer.

14. Procédé cosmétique pour une substance kératinique, telle que la peau, comprenant l'étape consistant à :
appliquer la composition selon l'une quelconque des revendications 1 à 13 sur la substance kératinique.

15. Utilisation d'une combinaison de :
(a) au moins un amidon modifié ;
(b) au moins un acide gras en C₁₃ à C₁₅ ; et
(d) au moins un tensioactif amphotère,
dans laquelle
le rapport pondéral de la quantité du tensioactif amphotère (d)/quantité de l'acide gras en C₁₃ à C₁₅ (b) est supérieur ou égal à 0,5, de préférence supérieur ou égal à 0,6, et plus préférablement supérieur ou égal à 0,7,
afin d'augmenter le dépôt sur une substance kératinique, telle que la peau, d'au moins une argile (c).
